# EUROPEAN PATENT SPECIFICATION

(11) **EP 3 903 911 B1**
(45) Date of publication and mention of the grant of the patent: **04.05.2022**
(21) Application number: 20382345.5
(22) Date of filing: 28.04.2020
(51) Int. Cl.: B01D 53/62, B01J 27/18, C01B 25/32, C07C 29/00, C07C 31/02, C10L 1/02, C12P 7/06, E21B 41/00, C07C 29/36, B01J 21/06, B01J 35/00, B01J 37/02, B01J 37/08, B01J 37/34

(54) **PROCESS FOR PRODUCING ETHANOL**
VERFAHREN ZUR HERSTELLUNG VON ETHANOL
PROCÉDÉ DE PRODUCTION D'ÉTHANOL

(43) Date of publication of application: 03.11.2021
(73) Proprietor: B. Braun Surgical, S.A., 08191 Rubi (Barcelona) (ES); Universitat Politècnica de Catalunya, 08034 Barcelona (ES)
(72) Inventor: Turón Dols, Pau, 08191 Rubi (Barcelona) (ES); Sanz Beltrán, Vanesa, 08191 Rubi (Barcelona) (ES); Rodríguez Rivero, Anna M., 08191 Rubi (Barcelona) (ES); Alemán Llansó, Carlos, 08019 Barcelona (ES); Puiggalí Bellalta, Jordi, 08019 Barcelona (ES); Revilla-López, Guillem, 08019 Barcelona (ES); Sans, Jordi, 08019 Barcelona (ES)
(74) Representative: Patentanwälte Ruff, Wilhelm, Beier, Dauster & Partner mbB

(56) References cited:
- EP-A1- 3 278 818
- EP-A1- 3 321 230
- US-A1- 2008 220 489
- US-A1- 2010 105 118

## Description

### FIELD OF THE INVENTION

The present invention relates to a process for producing, in particular selectively producing, ethanol and to the use of the process for removing carbon dioxide (CO₂) from the atmosphere.

### BACKGROUND OF THE INVENTION

Carbon dioxide (CO₂) is considered the primary greenhouse gas and the main cause for global climate warming. Therefore, the efficient utilization of it as C1 feedstock to synthesize valuable chemical and industrial products is drawing increasing attention. For example, it is known that carbon dioxide may be employed as C1 feedstock to synthesize ethanol (B. An, Z. Li, Y. Song, J. Z. Zhang, L. Z. Zeng, C. Wang, W. B. Lin, Natur. Catal. 2019, 2, 709-717; C. Liu, B. C. Colon, M. Ciesack, P. A. Silver, D. G. Nocera, Science 2016, 352, 1210-1213; D. Wang, Q. Y. Bi, G. H. Yin, W. L. Zhao, F. Q. Huang, X. M. Xie, M. H. Jiang, Chem. Commun. 2016, 52, 14226-14229).

Further, it is known that transition metals and complexes that can act as transition metals dominate the catalysis associated to CO₂ activation and fixation (C. S. Yeung, Angew. Chem. Int. Ed. 2019, 58, 5492-5502; C. Weetman, S. Inoue, ChemCatChem 2018, 10, 4213-4228; P. P. Power, Nature 2010 463 171-177; D. D. Zhu, J. L. Liu, S. Z. Adv. Mater. 2016, 28, 3423-3452).

A process for the production of C2+ alcohols, namely ethanol and heavier alcohols, from a methane-containing feedstock is known from US 2010/0105118 A1. A method for producing methanol and higher alcohols, in particular ethanol, from biological wastes is known from US 2008/0220489 A1.

Permanently polarized hydroxyapatite, a process for its manufacture and uses of permanently polarized hydroxyapatite is known from EP 3 278 818 A1 and EP 3 321 230 A1.

The synthesis of ethanol often lacks from the drawback that the synthesis requires harsh reaction conditions and, in particular due to the catalyst, is environmentally unfriendly, complicated and expensive.

### OBJECT AND SOLUTION

In view of the foregoing, the object underlying the present invention is therefore to make available a process for producing, in particular selectively producing, ethanol which circumvents drawbacks, in particular as described above, in the context of conventional syntheses of ethanol.

This object is accomplished by a process according to independent claim 1 and by the use according to claim 15. Preferred embodiments of the process are defined in the dependent claims and the present description. The subject-matter and wording, respectively of all claims is hereby incorporated into the description by explicit reference.

The present invention relates to a process for producing or synthesizing, in particular selectively producing or synthesizing, ethanol. The process comprises the step of
- contacting a gas mixture comprising or consisting of carbon dioxide (CO₂) and methane (CH₄) in presence of water, in particular liquid water, (H₂O) with a catalyst comprising or consisting of permanently polarized hydroxyapatite.

Hereafter, the above step of the inventive process is denoted as "contacting step".

Preferably, the contacting step is carried out at room temperature.

The present invention rests on the surprising finding that production or synthesis, in particular selective production or synthesis, of ethanol from carbon dioxide and methane in the presence of permanently polarized hydroxyapatite as catalyst is achievable under mild conditions (particularly < 10 bar pressure and < 250 °C temperature) with lower levels of environmental contamination and cost. Without wishing to be bound by theory, the production or synthesis of ethanol according to the present invention involves hydrogenation of reduced carbon dioxide and C-C bond construction. Thus, the process according to the present invention may also be denoted as electro-reduction process of carbon dioxide towards ethanol and the permanently polarized hydroxyapatite may also be denoted as electro-catalyst.

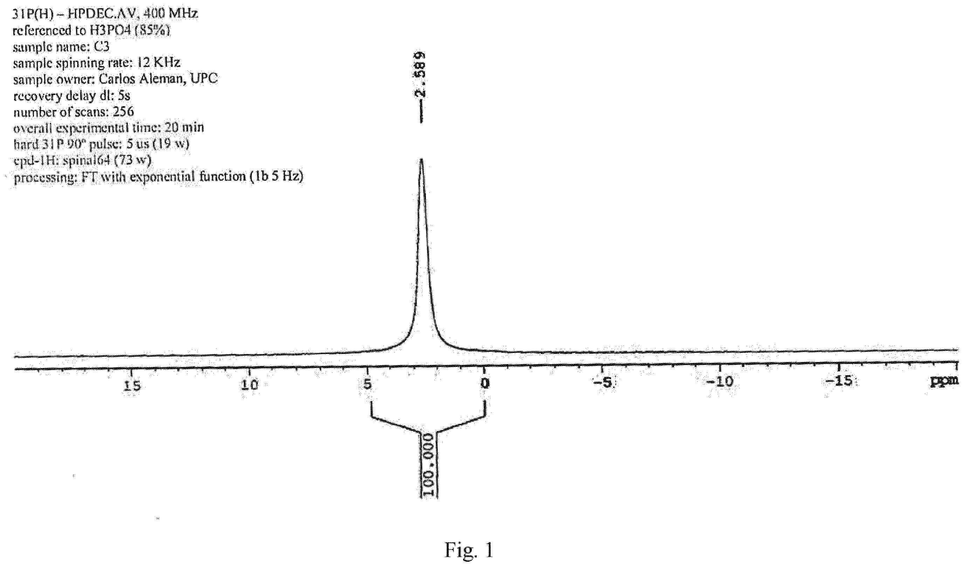

The term "permanently polarized hydroxyapatite" as used according to the present invention means a hydroxyapatite that has undergone a complete structural redistribution, in particular almost perfect, with a high crystallinity degree, i.e. particularly with a low amount of amorphous calcium phosphate and the presence of vacancies detected by increased electrochemical activity and the accumulation of charge per unit mass and surface. It has an electrochemical activity and ionic mobility which do not disappear over. The corresponding ³¹P-NMR spectrum of the permanently polarized hydroxyapatite is as shown on fig. 1.

The term "thermally polarized hydroxyapatite" as used according to the present invention preferably means a permanently polarized hydroxyapatite obtained or obtainable by a process (thermal polarization process) comprising the steps of
(a) sintering samples of hydroxyapatite, in particular at a temperature between 700°C and 1200°C, and
(b) applying a constant or variable DC voltage, in particular between 250 V and 2500 V for at least 1 min at a temperature between 900 °C and 1200 °C, or
   applying an equivalent field, in particular between 1.49 kV/cm and 15 kV/cm for at least 1 min at a temperature between 900 °C and 1200 °C, or
   applying an electrostatic discharge, in particular between 2500 V and 1500000 V for less than 10 min at a temperature between 900 °C and 1200 °C, or
   applying an equivalent electrical field, in particular between 148.9 kV/cm and 8928 kV/cm for less than 10 min at a temperature between 900 °C and 1200 °C.

The term "room temperature" as used according to the present invention means a temperature from 15 °C to 35 °C, in particular 18 °C to 30 °C, preferably 20 °C to 30 °C, more preferably 20 °C to 28 °C, particularly 20 °C to 25 °C.

In an embodiment of the invention, the permanently polarized hydroxyapatite comprises or has
- a crystallinity > 65 %, in particular > 70 %, preferably > 75 %, more preferably from 65 % to 99.9 %,
   and/or
- a proportion of amorphous calcium phosphate < 18 %, in particular from 0.1 % to 17 % or < 9 %, preferably < 5 %, in particular < 0.1 %, based on the total weight of the permanently polarized hydroxyapatite,
   and/or

- a proportion of β-tricalcium phosphate < 36 %, in particular from 0.1 % to 35 % or < 12 %, preferably < 5 %, in particular < 0.5 %, based on the total weight of the permanently polarized hydroxyapatite,
   and/or
- a bulk resistance from 10⁷ Ω cm² to 10⁴ Ω cm², in particular 10⁷ Ω cm² to 10⁵ Ω cm², in particular 10⁶ Ω cm² to 10⁵ Ω cm², preferably of 10⁵ Ω cm²,
   and/or
- a surface capacitance which decreases less than 8%, in particular from 8 % to 0.1 %, preferably from 5 % to 3 %, after 3 months.

The term "bulk resistance" as used according to the present invention means resistance to the electron transfer and may be determined by means of electrochemical impedance spectroscopy.

Preferably, the bulk resistance increases by only 0.1 % to 33 %, in particular 4 % to 63 %, preferably by 4 %, after 3 months.

The term "surface capacitance" as used according to the present invention means capacitance attributed to surface changes of hydroxyapatite induced by a thermal polarization process and may be determined by means of electrochemical impedance spectroscopy.

With respect to further features and advantages of the permanently polarized hydroxyapatite as used according to the present invention, it is referred to the PCT application WO 2018/024727 A1, the content of which is incorporated hereby by explicit reference.

In a further embodiment of the invention, the permanently polarized hydroxyapatite is obtained or obtainable by a process comprising the steps of
(a) preparing samples of hydroxyapatite, in particular crystalline hydroxyapatite,
(b) sintering the samples prepared in step (a), in particular at a temperature between 700 °C and 1200 °C,
(c) applying a constant or variable DC voltage, in particular between 250 V and 2500 V for at least 1 min at a temperature between 900 °C and 1200 °C, or
   applying an equivalent electric field, in particular between 1.49 kV/cm and 15 kV/cm for at least 1 min at a temperature between 900 °C and 1200 °C, or
   applying an electrostatic discharge, in particular between 2500 V and 1500000 V for less than 10 min at a temperature between 900 °C and 1200 °C, or
   applying an equivalent electrical field, in particular between 148.9 kV/cm and 8928 kV/cm for less than 10 min at a temperature between 900 °C and 1200 °C, and
(d) cooling the samples obtained in step (c) maintaining the DC voltage or the equivalent electric field or
   cooling the samples obtained in step (c) maintaining or without maintaining the electrostatic discharge or the equivalent electric field.

The aforementioned step (a) may be carried out by using ammonium phosphate dibasic (diammonium hydrogen phosphate, (NH₄)2HPO₄) and calcium nitrate (Ca(NO₃)₂) as reactants or starting materials. In particular, the step (a) may be carried out by
(a₁) providing a mixture, in particular an aqueous-alcoholic mixture, of ammonium phosphate dibasic and calcium nitrate,
(a₂) stirring the mixture provided in step (a₁), in particular at room temperature,
(a₃) hydrothermal treating of the mixture stirred in step (a₂),
(a₄) cooling the mixture hydrothermally treated in step (a₃),
(a₅) separating precipitates obtained after cooling the mixture in step (a₄), and
(a₆) freeze-drying the precipitates separated in step (a₅) to produce hydroxyapatite, in particular crystalline hydroxyapatite.

The term "room temperature" as used according to the present invention preferably refers to a temperature of 15 °C to 30 °C, in particular 18 °C to 25 °C, preferably 20 °C to 25 °C.

The step (a₁) may be in particular carried out by using a mixture comprising or consisting of ammonium phosphate dibasic, calcium nitrate, water, in particular de-ionized water, ethanol, and optionally chelated calcium solutions. Advantageously, the pH value of the mixture and/or the pH value of an aqueous calcium nitrate solution applied for providing the mixture may be adjusted to 10-12, preferably 10.5. Thus, shapes and sizes of hydroxyapatite, in particular in the form of nanoparticles, can be controlled. Further, the step (a₂) may be carried out under agitation, in particular gentle agitation, for example applying 150 rpm to 400 rpm. Further, the step (a₂) may be carried out for 1 min to 12 h, in particular for 1 h. The step (a₂) may also be termed as an aging step, according to the present invention. Further, the step (a₃) may be carried out at a temperature of 60 °C to 240 °C, preferably of 150 °C. Further, the step (a₃) may be carried out at a pressure of 1 bar to 250 bar, preferably of 20 bar. Further, the step (a₃) may be carried out for 0.1 h to 72 h, preferably for 24 h. Further, the step (a₄) may be carried out by cooling the mixture hydrothermally treated in step (a₃) to a temperature of 0 °C to 90 °C, in particular of 25 °C. Further, the step (a₅) may be carried out by means of centrifugation and/or filtration. Further, the precipitates separated in step (a₅) may be washed, in particular using water and/or a mixture of ethanol and water, before the step (a₆) is carried out. Further, the step (a₆) may be carried out for 1 day to 4 days, in particular for 2 days to 3 days, preferably for 3 days.

Further, the aforementioned step (b) may be carried out at a temperature between 700 °C and 1150 °C, in particular between 800 °C and 1100 °C, in particular at 1000 °C.

Further, the constant or variable DC voltage or the equivalent electric field may be applied in the aforementioned step (c) for 1 h to 24 h, in particular 0.1 h to 10 h, in particular 1 h.

Further, the DC voltage applied in the aforementioned step (c) is preferably 500 V, which is equivalent to a constant electric field of 3 kV/cm.

Further, the equivalent electric field applied in the aforementioned step (c) is preferably 3 kV/cm.

Further, the temperature in step (c) is preferably at least 900 °C, more preferably 900 °C to 1000 °C, in particular 1000 °C.

Further, the process for obtaining the permanently polarized hydroxyapatite may comprise between the step (b) and the step (c) a further step
(bc) pressing the samples obtained in step (b), in particular at 620 MPa and/or for 10 min, to produce discs of sintered samples of hydroxyapatite, in particular crystalline hydroxyapatite, and subsequently sandwiching the discs of sintered samples of hydroxyapatite, in particular crystalline hydroxyapatite, between plates, in particular made of stainless steel.

Further, the aforementioned step (d) may be carried out by cooling the samples obtained in step (c) to room temperature.

Further, the aforementioned step (d) may be carried out for 1 min to 72 h, in particular 15 min to 5 h, preferably 15 min to 2 h.

In a further embodiment of the invention, the permanently polarized hydroxyapatite is obtained or obtainable by a process comprising the steps of
(a) preparing samples of hydroxyapatite, in particular crystalline hydroxyapatite, in particular using ammonium phosphate dibasic (diammonium hydrogen phosphate, (NH₄)2HPO₄) and calcium nitrate (Ca(NO₃)₂) as reactants or starting materials,
(b) sintering the samples prepared in step (a), in particular at a temperature of 1000 °C, in particular for 2 h,
(c) applying an equivalent electric field of 3 kV/cm, in particular at a temperature of 1000 °C, in particular for 1 h, and
(d) cooling the samples obtained in step (c) maintaining the equivalent electric field, in particular for 30 min.

In a further embodiment of the invention, the contacting step is carried out with a volumetric ratio of permanently polarized hydroxyapatite to water, in particular liquid water, of 1000:1 to 0.01:1, in particular 500:1 to 100:1, preferably 300:1 to 350:1.

In a further embodiment of the invention, the contacting step is carried out with a volumetric ratio of carbon dioxide to methane of 200:1, in particular 3:1, preferably 1:1.

In a further embodiment of the invention, the contacting step is carried out under a total pressure of 0.1 bar to 100 bar, in particular 0.1 bar to 10 bar, in particular 1 bar to 10 bar, in particular 1 bar to 8 bar, in particular 1 bar to 6 bar, preferably of 6 bar.

The term "total pressure" as used according to the present invention refers to the sum of each gas' partial pressure of the gas mixture, preferably at room temperature.

In a further embodiment of the invention, the contacting step is carried out under a partial pressure of carbon dioxide of 0.035 bar to 90 bar, in particular 0.1 bar to 3 bar, in particular 1 bar to 3 bar, preferably of 3 bar, and/or under a partial pressure of methane of 0.00017 bar to 5 bar, in particular 1 bar to 3 bar, preferably of 3 bar.

Further, the contacting step may be carried out with a total pressure of the gas mixture from 0.0001 bar to 250 bar in the presence of the catalyst and the water, in particular liquid water.

Further, the contacting step may be carried out with a pressure ratio of carbon dioxide to methane (CO₂ : CH₄) in the presence of the catalyst from 0.0001 bar : 250 bar to 250 bar : 0.0001 bar.

Further, the gas mixture may additionally comprise nitrogen (N₂). In particular, the contacting step may be carried out with a pressure ratio of carbon dioxide to nitrogen (CO₂ : N₂) in the presence of the catalyst from 0.0001 bar : 250 bar to 250 bar : 0.0001 bar. Further, the contacting step may be carried out with a pressure ratio of carbon dioxide to a mixture of methane and nitrogen (CO₂ : mixture of CH₄ and N₂) in the presence of the catalyst from 0.0001 bar : 250 bar to 250 bar : 0.0001 bar.

Preferably, the contacting step is carried out by using an uncoated permanently polarized hydroxyapatite, i.e. by using a permanently polarized hydroxyapatite lacking any coating. In that regard, it surprisingly turned out that the application of an uncoated permanently polarized hydroxyapatite advantageously significantly increases the conversion of carbon dioxide and methane into ethanol and in addition maximizes the selective synthesis of ethanol as the major reaction product.

Alternatively, the contacting step may be carried out by using a coated permanently polarized hydroxyapatite. In principle, the contacting step may be carried out by using a permanently polarized hydroxyapatite being coated with an inorganic photocatalyst such as TiO₂, MgO₂, MnO₂ or combinations thereof. More specifically, the contacting step may be carried out by using a permanently polarized hydroxyapatite having a three-layered coating, in particular wherein the three-layered coating may be composed of two layers of aminotris(methylenephosphonic acid) and a layer of zirconium oxychloride (ZrOCl₂) or zirconia (ZrO₂), wherein the layer of zirconium oxychloride is arranged or sandwiched between the two layers of aminotris(methylenephosphonic acid). By using a coated permanently polarized hydroxyapatite, the efficiency of the reaction can be advantageously increased.

In a further embodiment of the invention, the contacting step is carried out under UV (ultraviolet) irradiation or UV-Vis (ultraviolet-visible) irradiation. In particular, the contacting step may be carried out under UV irradiation or UV-Vis irradiation having a wavelength from 200 nm to 850 nm, in particular 240 nm to 400 nm, preferably 250 nm to 260 nm, more preferably of 253.7 nm. Further, the contacting step may be in particular carried out under UV irradiation having a wavelength from 200 nm to 280 nm, in particular 240 nm to 270 nm, preferably 250 nm to 260 nm, more preferably of 253.7 nm. Preferably, the permanently polarized hydroxyapatite is directly exposed to or irradiated with the UV irradiation or UV-Vis irradiation. Advantageously, the UV irradiation or UV-Vis irradiation are/is provided by a suitable UV source and/or Vis source, for example UV lamp and/or Vis lamp.

In a further embodiment of the invention, the contacting step is carried out under UV (ultraviolet) irradiation or UV-Vis (ultraviolet-visible) irradiation having a irradiance from 0.1 W/m² to 200 W/m², in particular 1 W/m² to 50 W/m², preferably 2 W/m² to 10 W/m², more preferably of 3 W/m². With respect to advantages of this embodiment, reference is made to the preceding paragraph.

In a further embodiment of the invention, the contacting step is carried out at a temperature of 25 °C to 250 °C, in particular 95 °C to 140 °C, preferably of 95 °C.

More preferably, the contacting step is carried out at a temperature of 95 °C and under UV irradiation. These reaction conditions are especially useful for accomplishing a highly selective synthesis of ethanol.

In a further embodiment of the invention, the contacting step is carried out without UV irradiation and at a temperature of 25 °C to 250 °C, in particular 95 °C to 140 °C, preferably of 140 °C.

Also the reaction conditions according this embodiment result in a highly selective synthesis of ethanol as the major reaction product.

In a further embodiment of the invention, the contacting step is carried out for 0.0001 h to 120 h, in particular 24 h to 72 h, preferably 48 h to 72 h.

Further, the process, in particular the contacting step, may be carried out continuously or discontinuously, in particular as a batch process.

Further, the contacting step may be carried out by using air, in particular traffic contaminated air, as gas mixture. Thus, it is possible to synthesize ethanol as a valuable compound and concurrently to remove carbon dioxide from air, in particular traffic contaminated air.

Further, the present invention relates to the use of the process according to the present invention for removing carbon dioxide from the air or atmosphere. With respect to further features and advantages of the use, reference is made in its entirety to the previous description.

Further features and advantages of the invention will become clear from the following examples in conjunction with the subject-matter of the dependent claims. The individual features can be realized either singularly or severally in combination in one embodiment of the invention. The preferred embodiments merely serve for illustration and better understanding of the invention and are not to be understood as in any way limiting the invention.

### DESCRIPTION OF THE FIGURES

For a better understanding of what has been disclosed, some figures are attached which schematically or graphically and solely by way of non-limiting example show a practical case of embodiment of the present invention.
Fig. 1 graphically shows a ³¹P-NMR spectrum of the permanently polarized hydroxyapatite (p-HAp) according to the present invention.
Fig. 2(a) schematically depicts the Raman spectra of hydroxyapatite (HAp) samples with the deconvolution of the v₁ peak in the 930-990 cm⁻¹ interval. Counts (A.U.) are plotted on the ordinate. Raman shift (cm⁻¹) is plotted on the abscissa.
Fig. 2(b) schematically depicts the Raman spectra of permanently polarized hydroxyapatite (p-HAp) samples with the deconvolution of the v₁ peak in the 930-990 cm⁻¹ interval. Counts (A.U.) are plotted on the ordinate. Raman shift (cm⁻¹) is plotted on the abscissa.

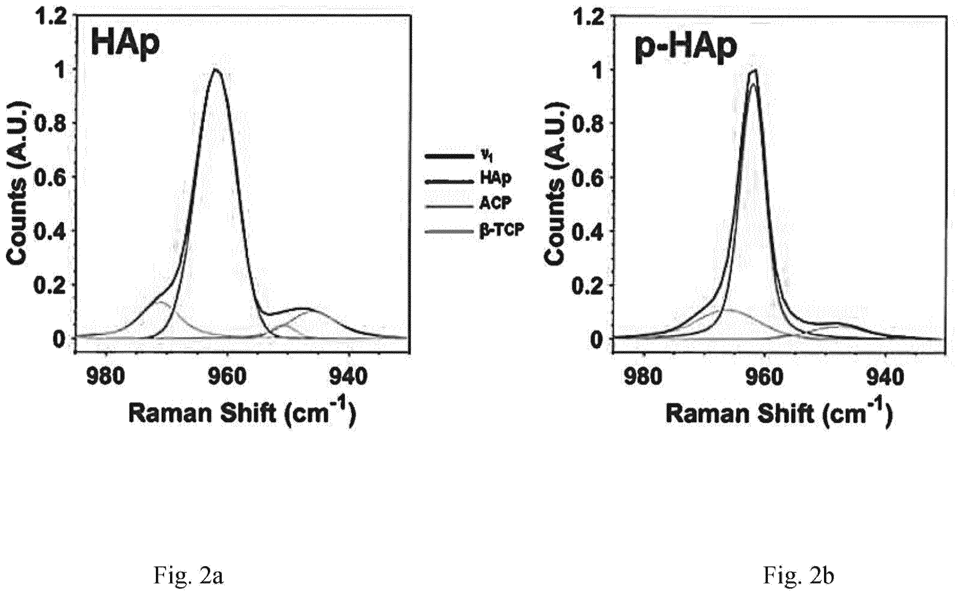

   The Figs. 2a-b, which compare the Raman v₁ peak in the 930-990 cm⁻¹ interval before and after polarization of Hap, prove the success of the polarization process.
   The area of HAp, amorphous calcium phosphate (ACP) and β-tricalcium phosphate (β-TCP) indicates the content of each phase. The content of co-existing phases experiences a reduction in polarized samples (i.e. 4.3 % and 9.8 % for ACP and β-TCP, respectively) that is accompanied by a decrease of the full width at half maximum (FWHM) from 9 cm⁻¹ in HAp to 5 cm⁻¹ in p-HAp. This result indicates an increase of the HAp phase by means of a reduction of crystal imperfections, such as PO₄³⁻ tetrahedrons distortions.

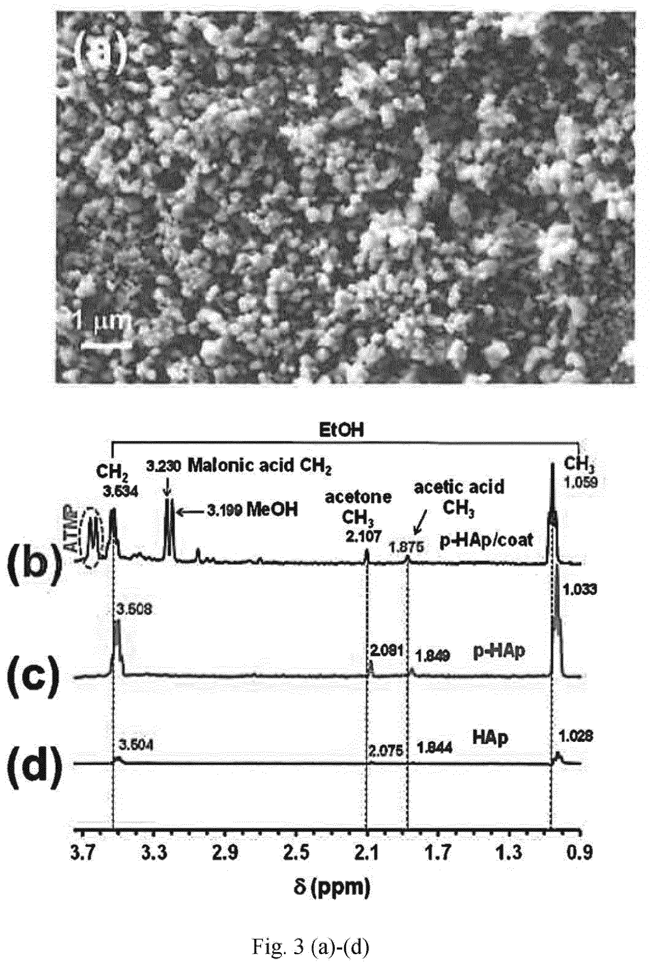

Fig. 3(a) shows a scanning electron microscopy micrograph of the permanently polarized hydroxyapatite. Accordingly, the permanently polarized hydroxyapatite can be described as particles of (approximately) 100 nm to 300 nm that aggregate forming agglomerates of up to 1 µm in size.
Fig. 3(b) graphically shows the ¹H-NMR spectrum of a solution obtained after extraction of the reaction products of a reaction that was conducted for 72 h using CO₂ (3 bar), CH₄ (3 bar), H₂O (1 mL) in the presence of coated p-HAp. The p-HAp was coated with aminotris(methylenephosphonic acid), hereafter denoted as ATMP, and zirconium oxychloride (ZrOCl₂), hereafter denoted as ZC, at 95 °C under UV radiation. The reacted catalyst was dissolved in an aqueous solution with 100 mM HCI and 50 mM NaCl.
Fig. 3(c) graphically shows the ¹H-NMR spectrum of a solution obtained after extraction of the reaction products of a reaction that was conducted for 72 h using CO₂ (3 bar), CH₄ (3 bar), H₂O (1 mL) using (uncoated) permanently polarized hydroxyapatite as catalyst, at 95 °C under UV radiation. The reacted catalyst was dissolved in an aqueous solution with 100 mM HCI and 50 mM NaCl.
Fig. 3(d) graphically shows the ¹H-NMR spectrum of a solution obtained after extraction of the reaction products of a reaction that was conducted for 72 h using CO₂ (3 bar), CH₄ (3 bar) and H₂O (1 mL) using conventional (i.e. non-polarized) hydroxyapatite as a catalyst, at 95 °C under UV radiation. The reacted catalyst was dissolved in an aqueous solution with 100 mM HCI and 50 mM NaCl.
   As is shown in figs. 3(b)-(d), chemical shifts observed after the dissolution of the coated p-HAp are slightly deshielded with respect to the peaks of products derived from non-coated catalysts. This effect has been attributed to the aminotris(methylenephosphonic acid) (ATMP), which increases the acidity of the medium, causing downfield shifts that are not detected for p-HAp and HAp, independently of the conditions. On the other hand, figs. 3(b)-(c) indicate that the elimination of the coating from the catalyst not only increases the conversion into ethanol by 20 %, but also maximizes the selective synthesis of ethanol as the major reaction product (from 67 % to 93 %).

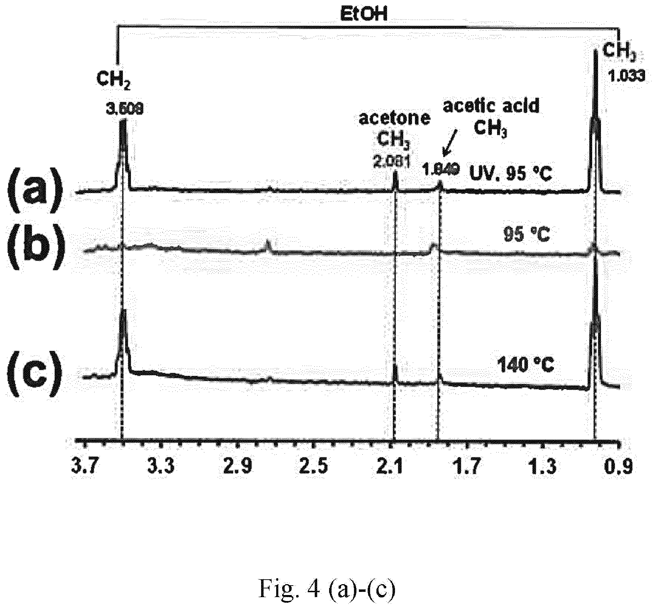

Fig. 4(a) graphically shows a further ¹H-NMR spectrum of a solution obtained after extraction of the reaction products achieved after 72 h from CO₂ (3 bar), CH₄ (3 bar) and H₂O (1 mL) using (uncoated) permanently polarized hydroxyapatite as catalyst and 95 °C and UV radiation as reaction conditions. The catalyst was dissolved in an aqueous solution with 100 mM HCI and 50 mM NaCl.
Fig. 4(b) graphically shows the ¹H-NMR spectrum of a solution obtained after extraction of the reaction products achieved after 72 h from CO₂ (3 bar), CH₄ (3 bar) and H₂O (1 mL) using (uncoated) permanently polarized hydroxyapatite as catalyst and 95 °C without UV radiation as reaction conditions. The catalyst was dissolved in an aqueous solution with 100 mM HCI and 50 mM NaCl.
Fig. 4(c) graphically shows the ¹H-NMR spectrum of a solution obtained after extraction of the reaction products achieved after 72 h from CO₂ (3 bar), CH₄ (3 bar) and H₂O (1 mL) using permanently polarized hydroxyapatite as catalyst and 140 °C without UV radiation as reaction conditions. The catalyst was dissolved in an aqueous solution with 100 mM HCI and 50 mM NaCl.

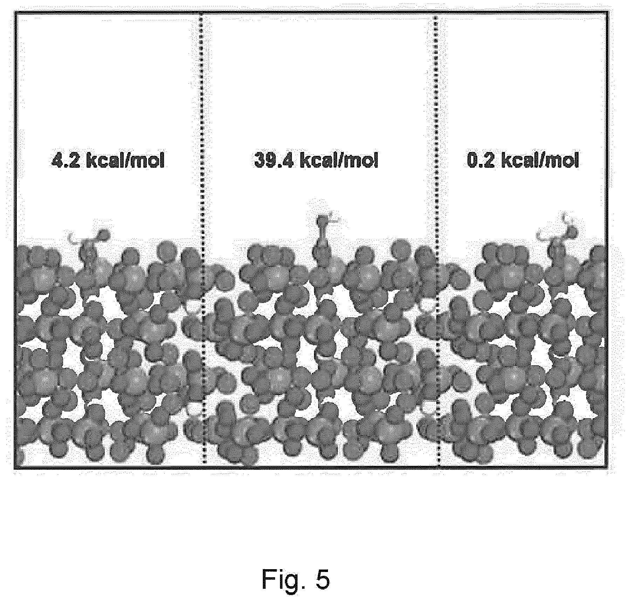

Fig. 5 graphically shows representation of three protonated forms of CO₂ adsorbed molecules on the OH⁻ vacancy of permanently polarized hydroxyapatite. The numeric values (in eV) stand for the calculated adsorption energies.
   In order to support the p-HAp fixation mechanism based on the formation of carboxylates, DFT calculations were performed at the PBE-D3 level. Calculations were performed considering the (0001) facet, which is the most stable HAp surface, and considering an isodesmic model in which H₂ is used as a source of protons. The adsorption energies of three different protonation products of CO₂ were calculated by inserting the molecules in the hydroxyl vacancy of the mineral. Results proved that the protonation of CO₂ to formic acid is exothermic in the gas phase by -3.1 kcal/mol, but it is more exothermic when adsorbed on p-HAp substrate, by -32.7 kcal/mol. Yet, all protonated species display endothermic adsorption energies, the one for the protonated formic acid is very small (0.2 kcal/mol) while the one for the CO₂ is 5.1 kcal/mol (other sites were checked on the p-HAp displaying higher energies, as shown for some representative cases in fig. 5), thus making this pathway unfeasible to be followed completely and shifting the catalysis location elsewhere close to.

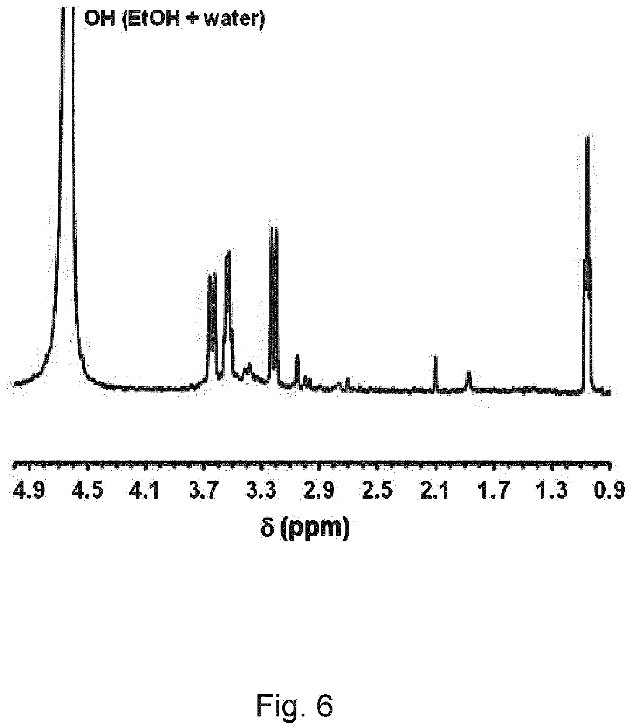

Fig. 6 graphically shows a further ¹H-NMR spectrum of a solution obtained after extraction of the reaction products achieved after 72 h from CO₂ (3 bar), CH₄ (3 bar) and H₂O (1 mL) in the presence of permanently polarized hydroxyapatite coated with aminotris(methylenephosphonic acid) and zirconium oxychloride, at 95 °C under UV radiation. The reacted catalyst was dissolved in an aqueous solution with 100 mM HCI and 50 mM NaCl. The spectrum includes the OH band at 4.65 ppm.

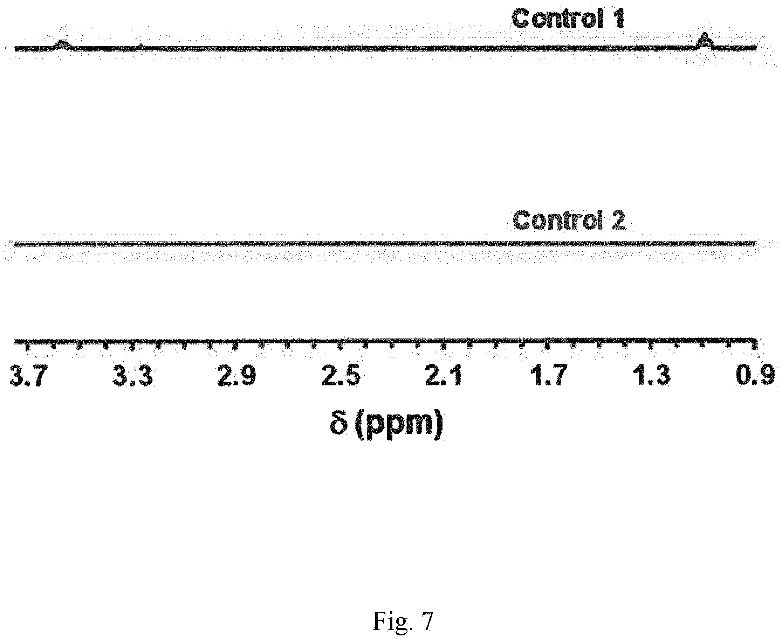

Fig. 7 graphically shows the ¹H-NMR spectrum of the liquid water after 72 h from CO₂ (3 bar), CH₄ (3 bar) and H₂O (1 mL) at 95 °C using UV radiation (control 1) and without using UV radiation (control 2). No catalyst was used for this reaction.

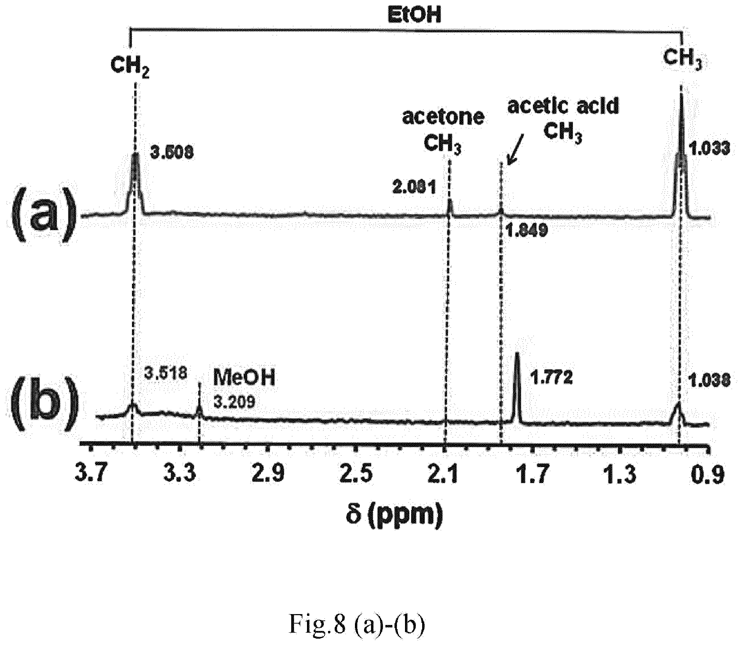

Fig. 8(a) shows a further ¹H-NMR spectrum of the reaction products achieved after 72 h from CO₂ (3 bar), CH₄ (3 bar) and H₂O (1 mL) at 95 °C using UV radiation and (uncoated) permanently polarized hydroxyapatite as catalyst, wherein the analysis of the solution obtained after extraction of the products was performed by dissolving the catalyst with 100 mM HCI and 50 mM NaCl.
Fig. 8(b) graphically shows the ¹H-NMR spectra of the reaction products achieved after 72 h from CO₂ (3 bar), CH₄ (3 bar) and H₂O (1 mL) at 95 °C using UV radiation and using (uncoated) permanently polarized hydroxyapatite as catalyst, wherein liquid water, which has been incorporated to the reaction chamber, has been analyzed.

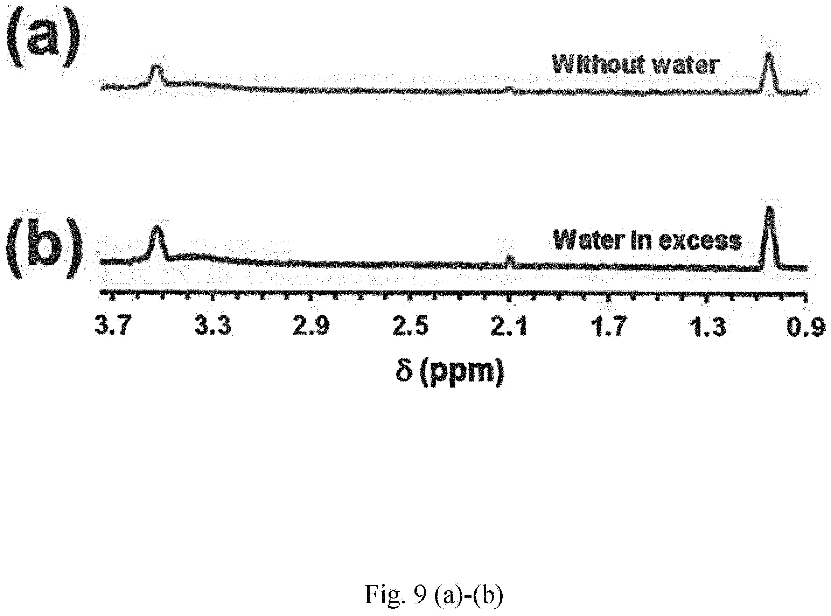

Fig. 9(a) graphically shows the ¹H-NMR spectrum of the reaction products achieved after 72 h from CO₂ (3 bar) and CH₄ (3 bar) at 95 °C using UV radiation and (uncoated) permanently polarized hydroxyapatite as catalyst in absence of water.
Fig. 9(b) graphically shows the ¹H-NMR spectrum of the reaction products achieved after 72 h from CO₂ (3 bar) and CH₄ (3 bar) at 95 °C using UV radiation and (uncoated) permanently polarized hydroxyapatite as catalyst with an excess of water.

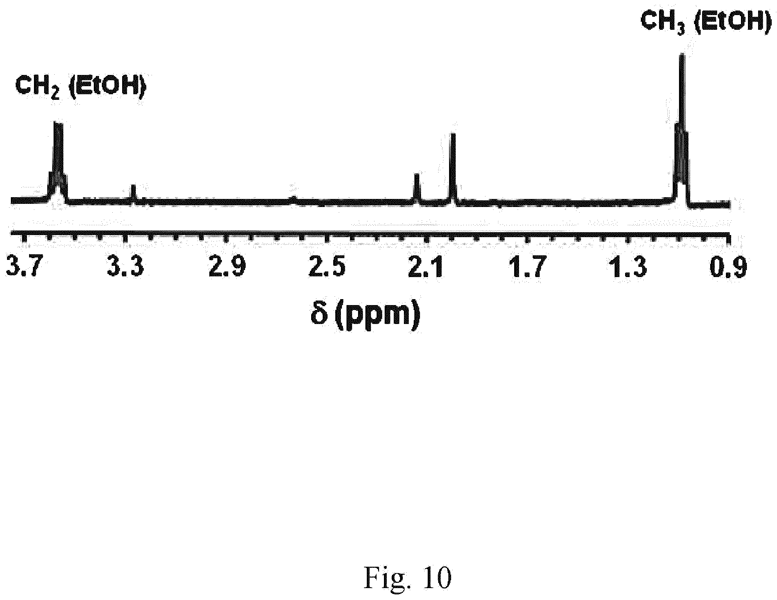

Fig. 10 graphically shows the ¹H-NMR spectrum of the solution obtained after extraction of the reaction products achieved after 72 h from polluted air (atmospheric pressure) and H₂O (1 mL) at 95 °C using (uncoated) permanently polarized hydroxyapatite as catalyst at 95 °C and under UV radiation. The reacted catalyst was dissolved in an aqueous solution with 100 mM HCI and 50 mM NaCl.

### EXPERIMENTAL SECTION

### 1. Materials

Calcium nitrate (Ca(NO₃)₂), diammonium hydrogen phosphate ((NH4)₂HPO₄; purity > 99.0 %) and ammonium hydroxide solution 30 % (NH₄OH; purity: 28-30% w/w) were purchased from Sigma Aldrich. Ethanol (purity > 99.5 %) was purchased from Scharlab. All experiments were performed with milli-Q water.

### 2. Hydrothermal synthesis of hydroxyapatite (HAp)

15 mL of 0.5 M of NH4)₂HPO₄ in de-ionized water were added at a rate of 2 mL min⁻¹ to 25 mL of 0.5 M of Ca(NO₃)₂ in ethanol (with pH previously adjusted to 10.5 using ammonium hydroxide solution) and left aging for 1 h. The whole process was performed under gentle agitation (150 rpm) and at room temperature. Hydrothermal treatment at 150 °C was applied using an autoclave Digestec DAB-2 for 24 h. The autoclave was allowed to cool down before opening. The precipitates were separated by centrifugation and washed with water and a 60/40 v/v mixture of ethanol-water (twice). After freeze-drying it for three days, the white powder obtained was sintered for 2 h at 1000 °C in air using the Carbolite ELF11/6W/301 furnace.

### 3. Thermally stimulated polarization process (TSP)

Mechanical consistent discs of around 1.5 mm of thickness were obtained by pressing 150 mg of previously sintered HAp powder at 620 MPa for 10 min. Thermal polarization was done placing the HAp discs between two stainless steel (AISI 304) and applying 3 kV/cm at 1000 °C for 1 h with a GAMMA power supply at 1000 °C using the same laboratory furnace as mentioned above. The discs were allowed to cool down maintaining the applied electric potential for 30 minutes, and finally, all the system was powered off and left to cool overnight.

### 4. Characterization

Vibrational spectra for a structural fingerprint were obtained by the inVia Qontor confocal Raman microscope (Renishaw), equipped with a Renishaw Centrus 2957T2 detector and a 785 nm laser.

SEM images were obtained using a Zeiss Neon 40 microscope equipped with a SEM GEMINI column. HRTEM was performed in a JEOL 2010F microscope equipped with a field emission electron source and operated at an accelerating voltage of 200 kV. The point-to-point resolution was 0.19 nm, and the resolution between lines was 0.14 nm. Samples were dispersed in an alcohol suspension in an ultrasonic bath, and a drop of the suspension was placed over a grid with holey-carbon film. Images were not filtered or treated by means of digital processing and they correspond to raw data. All ¹H-NMR spectra were acquired with a Bruker Avance III-400 spectrometer operating at 400.1 MHz and accumulating sixty-four scans. The chemical shift calibration was carried out using tetramethylsilane as internal standard. The samples were dissolved in milli-Q water containing 100 mM of HCI and 50 mM NaCl with the final addition of deuterated water.

### 5. Computational details

The 2 × 1 × 2 HAp supercell was chosen to build the (0001) facet for p-HAp. The latter was built by removing an OH⁻ orthonormal to the surface from the HAp supercell, which was previously optimized at the chosen DFT level. Consequently, a +1 global charge was applied for all calculations except for those involving formate, unpaired spin being considered when necessary. The initial coordinates of HAp were optimized following the computational details provided below to unwind surface tensions. The plane waves approach implemented in the Quantum Espresso 4.6 suite of Open-Source computer codes was used. Calculations were performed at the PBE level of theory corrected with the Grimme three body dispersion potentials (PBE-D3), applying the default C₆ software coefficients. A kinetic energy cutoff for the wave functions of 40 Ry was employed. A k-point mesh of 3 × 3 × 1 was automatically generated. Instead, a Gamma-center 1 × 1 × 1 k-mesh was used for calculations of discrete molecules and a 7 × 7 × 7 k-mesh for the bulk HAp calculations. Geometry optimizations were performed using the conjugated gradient algorithm until both the energy and force variation between consecutive steps was below 10⁻³ a.u and 10⁻⁴ a.u, respectively. The energy at each step was optimized until the deviation from self-consistency was below 10⁻⁵ Ry. Adsorption energies were calculated according to the following process: A+S → AS*, where A is the adsorbate; S the surface and AS* the adsorbed state. The adsorption energy (E_{ads}) was expressed as E_{ads}=E_{AS*} - (E_{A} + Eₛ).

### 6. Reaction chamber

A high pressure stainless steel reactor, which was designed ad hoc, was used to perform all the reactions. In brief, the reactor was dotted with a manometer, an electric heater with a thermocouple and an external temperature controller. The reactor was also characterized by an inert reaction chamber coated with a perfluorinated polymer (Teflon, 120 mL), where both the catalyst and water were incorporated. The reactor was equipped with three independent inlet valves for the incorporation of gases and an outlet valve to recover the gaseous reaction products. A UV lamp (GPH265T5L/4, 253.7 nm) was also placed in the middle of the reactor to irradiate the catalyst directly, the lamp being protected by a UV transparent quartz tube. All surfaces were coated with a thin film of perfluorinated polymer (Teflon) in order to avoid any contact between the reaction medium and the reactor surfaces, in this way discarding other catalyst effects.

### 7. Synthesis of coated p-HAp

Three-layered systems consisting of the successive deposition of aminotris(methylenephosphonic acid) (ATMP) and zirconium oxychloride (ZC) onto p-HAp were obtained by immersion in the corresponding aqueous solutions at room temperature for 5 h. In order to deposit a first ATMP layer, p-HAp was immersed into a 5 mM ATMP solution for 5 h. Subsequently, ZC was deposited onto the ATMP layered p-HAp by immersing the latter into a 5 mM ZrOCl₂ solution for 5 h. Finally, a second layer of ATMP was deposited on the ZC and ATMP layered p-HAp by immersing the latter into a 1.25 mM ATMP solution for 5 h.

### 8. Synthesis of ethanol using uncoated p-HAp as catalyst

Ethanol was synthesized from CO₂ and CH₄ gas mixture (3 bar each) in the presence of uncoated p-HAp as catalyst and in the presence of liquid H₂O (1 mL). The reaction was carried out for 72 h at 95 °C and under irradiation of an UV lamp (GPH265T5L/4, 253.7 nm) illuminating directly the uncoated p-HAp. The reaction resulted in the following yields (expressed as µmol of product per gram of catalyst): ethanol (19.4 ± 3.8 µmol/g), acetone (0.9 ± 0.1 µmol/g) and acetic acid (0.6 ± 0.1 µmol/g). This result represents a 20% increment in the yield of ethanol, while methanol and malonic acid were not detected.

### 9. Synthesis of ethanol using coated p-HAp as catalyst

Ethanol was synthesized from CO₂ and CH₄ gas mixture (3 bar each) in the presence of coated p-HAp as catalyst and in the presence of liquid H₂O (1 mL). The reaction was carried out for 72 h at 95 °C and under irradiation of an UV lamp (GPH265T5L/4, 253.7 nm) illuminating directly the coated p-HAp. The p-HA was coated with two layers of aminotris(methylenephosphonic acid) and a layer of zirconium oxychloride (ZrOCl₂), wherein the layer of zirconium oxychloride was arranged or sandwiched between the two layers of aminotris(methylenephosphonic acid). The reaction resulted in the following yields (expressed as µmol of product per gram of coated p-HAp): ethanol (16.1 ± 3.2 µmol/g), methanol (4.9 ± 1.0 µmol/g), malonic acid (1.6 ± 0.2 µmol/g), acetone (0.8 ± 0.2 µmol/g) and acetic acid (0.6 ± 0.1 µmol/g). The predominant product, ethanol, was identified by means of ¹H-NMR spectroscopy not only by the quartet (CH₂) and the triplet (CH₃) at 3.53 ppm and 1.06 ppm, respectively, but also by the intense OH peak at 4.65 ppm.

### 10. Synthesis of ethanol using (uncoated) HAp as catalyst

Ethanol was synthesized from CO₂ and CH₄ gas mixture (3 bar each) in the presence of (uncoated) HAp as catalyst and in the presence of liquid H₂O (1 mL). The reaction was carried out for 72 h at 95 °C and under irradiation of an UV lamp (GPH265T5L/4, 253.7 nm) illuminating directly the p-HAp. The reaction resulted in a very poor yield of ethanol (1.9 ± 0.5 µmol/g catalyst). Further, the yield of acetone and acetic acid was < 0.1 µmol/g catalyst.

### 11. Synthesis of ethanol without a solid support acting as catalyst

Ethanol was synthesized from CO₂ and CH₄ gas mixture (3 bar each) in the presence of (uncoated) HAp as catalyst and in the presence of liquid H₂O (1 mL). The reaction was carried out for 72 h at 95 °C and under irradiation of an UV lamp (GPH265T5L/4, 253.7 nm). In absence of any solid support acting as catalyst (see fig. 7(a)), the yield of ethanol is practically 0 (0.1 ± 0.05 µmol/g). Such a small amount, which has been attributed to eventual photo-induced CO₂ reduction and water splitting, completely disappears in absence of UV radiation (see 7(b)).

### 12. Synthesis of ethanol using traffic contaminated air

As a proof of concept the reaction for synthesizing ethanol was conducted at atmospheric pressure using contaminated air taken from the surrounding area of the UPC (Universitat Politècnica de Catalunya) east campus in Barcelona, an area heavily contaminated by car traffic, since it is in front of one of the main roads of the city. The contaminated air by combustion of fossil carburant contains significantly higher CO₂ and CH₄ than the average of the ambient air. The reaction was conducted using p-HAp as catalyst, in presence of 1 mL of water and at 95 °C with UV radiation. Analysis of the reaction products after 72 h showed ethanol among other products, some of them non-identified in previous reactions with controlled gas mixtures. Despite the fact that the amount of ethanol was very small (1.1 ± 0.2 µmol/g), it was found to be the predominant reaction product confirming the potential applicability of p-HAp as catalyst to regenerate contaminated air while obtaining ethanol as valuable product.

### 13. Further investigations in terms of the mechanism pathway

The selective ethanol formation might be associated to the photo- or the electro-catalytic reduction of CO₂. In order to explore the rule of the UV radiation and electrochemical activity imparted by the thermal stimulated polarization process in the reaction catalyzed by p-HAp, the process without UV illumination at 95 °C was repeated. As shown in figs. 4(a)-(b), the yield of ethanol decreased drastically, from 19.4 ± 3.8 to 0.2 ± 0.03 µmol/g, while acetone and acetic acid were not detected. By increasing the temperature to 140 °C, the yield of ethanol, acetone and acetic acid increased to 17.8 ± 1.8, 0.9 ± 0.2 and 0.7 ± 0.1 µmol/g catalyst, respectively, these values being very similar to those achieved by using UV radiation and 95 °C (see fig. 4(c)). This feature indicates that the electro-catalytic CO₂ conversion predominates over the photocatalytic one and, also, that UV radiation can be used to lower the free energy barrier to form intermediates once CO₂ is adsorbed onto the surface of the catalyst.

Analysis of the liquid water incorporated to the reaction chamber (see fig. 8(b)) has not only provided additional information on the total yield, but has also confirmed that the catalytic reaction takes place over the p-HAp surface, supporting the first step of the theoretically proposed pathway. Interestingly, additional small amounts of ethanol (0.7 ± 0.1 µmol/g) and acetic acid (2.0 ± 0.5 µmol/g), which probably converts into acetone on the catalyst surface, formic acid (1.9 ± 0.6 µmol/g) and methanol (1.5 ± 0.3 µmol/g) can be identified as well. This feature suggests that both the CO₂ activation and the formation of the reduced COO⁻intermediate do not occur onto the surface of the solid catalyst, but close to it, which reinforces the mechanism suggested by in silico experiments. Thus, when the intermediate incorporates to the water, which is in contact with a p-HAp catalyst, does not evolve towards ethanol, but protonates to yield formic acid. Finally, methanol can be originated as a result of the methyl radicals trapped in water or from formic acid. In any case, the impact of the reaction product dissolved in the water phase on the ethanol selectivity is negligible.

In order to get deeper information about the rule of water in the transformation of CO₂ into ethanol, the reaction with p-HAp at 95 °C and UV radiation was repeated considering the two following scenarios: i) complete absence of water; and ii) an excess of initial water (20 mL) in the reactor. In the latter case, the catalyst was partially immersed in water and, during the reaction, a water vapor rich atmosphere was expected. The results show that regulation of the initial amount of water is advantageous for the efficient reduction of CO₂ (see fig. 9). Although both ethanol and acetone were obtained in absence of water, their yield was low (4.7 ± 0.9 and 0.1 ± 0.06 µmol/g, respectively) in comparison to achieved when 1 mL of water was introduced in the reaction chamber. This difference has been attributed to the difficulties in generating and stabilizing active radical species in the catalyst when is dry (i.e. only with water absorbed from atmospheric moisture). On the other hand, the excess of water also causes a reduction in the yield of ethanol and acetone (7.0 ± 0.1 and 0.3 ± 0.07 µmol/g, respectively), which again are the only products that are formed. However, in this case the low yield has been associated with the fact that the water saturated atmosphere may gas exchange more difficult at the hydrophilic catalyst interface.

In summary, it could be confirmed the catalytic activity of p-HAp to convert gaseous CO₂ in ethanol following an electro-reduction mechanism. Both theoretical calculations and experiments under different reaction conditions provided important insights about the mechanism pathway. As a proof of concept, the proposed reaction has been successful in obtaining ethanol from road traffic contaminated air, opening an exciting new avenue to transform greenhouse gas emissions into valuable chemical products using a simple catalyst based on an earth-abundant mineral.

## Claims

1. A process for producing, in particular selectively producing, ethanol comprising the step of
- contacting a gas mixture comprising or consisting of carbon dioxide and methane in presence of water with a catalyst comprising or consisting of permanently polarized hydroxyapatite.

2. The process according to claim 1, **characterized in that** the permanently polarized hydroxyapatite has
- a crystallinity > 65 %, in particular > 70 %, preferably > 75 %, more preferably from 65 % to 99 %, and/or
- a proportion of amorphous calcium phosphate < 18 %, in particular from 0.1 % to 17 %, based on the total weight of the permanently polarized hydroxyapatite, and/or
- a proportion of β-tricalcium phosphate < 36 %, in particular from 0.1 % to 35 %, based on the total weight of the permanently polarized hydroxyapatite, and/or
- a bulk resistance from 10⁷ Ω cm² to 10⁵ Ω cm², in particular 10⁶ Ω cm² to 10⁵ Ω cm², wherein the bulk resistance preferably increases by only 4 % to 73 %, in particular 4 % to 63 %, preferably by 4%, after 3 months, and/or
- a surface capacitance decreasing less than 8%, in particular from 8 % to 0.1 %, preferably 5 % to 3 %, after 3 months.

3. The process according to claim 1 or 2, **characterized in that** the permanently polarized hydroxyapatite is obtained by a process comprising the steps of
(a) preparing samples of hydroxyapatite,
(b) sintering the samples prepared in step (a) at a temperature between 700 °C and 1200 °C,
(c) applying a constant or variable DC voltage between 250 V and 2500 V for at least 1 minute at a temperature between 900 °C and 1200 °C or
applying an equivalent electric field between 1.49 kV/cm and 15 kV/cm for at least 1 minute at a temperature between 900 °C and 1200 °C or
applying an electrostatic discharge between 2500 V and 1500000 V for less than 10 minutes at a temperature between 900 °C and 1200 °C or
applying an equivalent electric field between 148.9 kV/cm and 8928 kV/cm for less than 10 minutes at a temperature between 900 °C and 1200 °C, and
(d) cooling the samples obtained in step (c) maintaining the DC voltage or the equivalent electric field or
cooling the samples obtained in step (c) maintaining or without maintaining the electrostatic discharge or the equivalent electric field.

4. The process according to any of the preceding claims, **characterized in that** the permanently polarized hydroxyapatite is obtained by a process comprising the steps of
(a) preparing samples of hydroxyapatite,
(b) sintering the samples prepared in step (a) at a temperature of 1000 °C, in particular for 2 h,
(c) applying an equivalent electric field of 3 kV/cm at a temperature of 1000 °C, in particular for 1 h, and
(d) cooling the samples obtained in step (c) maintaining the equivalent electric field, in particular for 30 minutes.

5. The process according to any of the preceding claims, **characterized in that** the contacting step is carried out in the presence of liquid water.

6. The process according to any of the preceding claims, **characterized in that** the contacting step is carried out with a volumetric ratio of the permanently polarized hydroxyapatite to water, in particular liquid water, of 1000:1 to 0.01:1, in particular 500:1 to 100:1, preferably 300:1 to 350:1.

7. The process according to any of the preceding claims, **characterized in that** the contacting step is carried out with a volumetric ratio of carbon dioxide to methane of 200:1, in particular 3:1, preferably 1:1.

8. The process according to any of the preceding claims, **characterized in that** the contacting step is carried out under a total pressure of 0.1 bar to 100 bar, in particular 1 bar to 10 bar, preferably of 6 bar.

9. The process according to any of the preceding claims, **characterized in that** the contacting step is carried out under a partial pressure of carbon dioxide of 0.035 bar to 90 bar, in particular 1 bar to 3 bar, preferably of 3 bar, and/or under a partial pressure of methane of 0.00017 bar to 5 bar, in particular 1 bar to 3 bar, preferably of 3 bar.

10. The process according to any of the preceding claims, **characterized in that** the contacting step is carried out under UV irradiation or UV-Vis irradiation having a wavelength from 200 nm to 850 nm, in particular 240 nm to 400 nm, preferably 250 nm to 260 nm, more preferably of 253.7 nm.

11. The process according to any of the preceding claims, **characterized in that** the contacting step is carried out under UV irradiation and/or visible light irradiation, in particular having a irradiance from 0.1 W/m² to 200 W/m², in particular 1 W/m² to 50 W/m², preferably 2 W/m² to 10 W/m².

12. The process according to any of the preceding claims, **characterized in that** the contacting step is carried out at a temperature of 25 °C to 250 °C, in particular 95 °C to 140 °C, preferably of 95 °C.

13. The process according to any of the claims 1 to 10, **characterized in that** the contacting step is carried out without UV irradiation and at a temperature of 140 °C.

14. The process according to any of the preceding claims, **characterized in that** the contacting step is carried out continuously or discontinuously and/or for 0.0001 h to 120 h, in particular 24 h to 72 h, preferably 48 h to 72 h.

15. Use of a process according to any of the preceding claims for removing carbon dioxide from the atmosphere.

## Patentansprüche

1. Verfahren zur Herstellung, insbesondere gezielten Herstellung, von Ethanol umfassend den Schritt
- in Kontakt bringen eines Gasgemischs, das Kohlendioxid und Methan umfasst oder daraus gebildet ist, in Gegenwart von Wasser mit einem Katalysator, der permanent polarisiertes Hydroxyapatit umfasst oder daraus gebildet ist.

2. Verfahren nach Anspruch 1, **dadurch gekennzeichnet, dass** das permanent polarisierte Hydroxyapatit aufweist
- eine Kristallinität > 65 %, insbesondere > 70 %, bevorzugt > 75 %, besonders bevorzugt von 65 % bis 99 %, und/oder
- einen Anteil an amorphem Calciumphosphat < 18 %, insbesondere von 0,1 % bis 17 %, bezogen auf das Gesamtgewicht des permanent polarisierten Hydroxyapatits, und/oder
- einen Anteil an β-Tricalciumphosphat < 36 %, insbesondere von 0,1 % bis 35 %, bezogen auf das Gesamtgewicht des permanent polarisierten Hydroxyapatits, und/oder
- einen Bulkwiderstand von 10⁷ Ω cm² bis 10⁵ Ω cm², insbesondere 10⁶ Ω cm² bis 10⁵ Ω cm², wobei der Bulkwiderstand nach 3 Monaten vorzugsweise nur um 4 % bis 73 %, insbesondere 4 % bis 63 %, bevorzugt um 4 % zunimmt, und/oder
- eine Oberflächenkapazität, die nach 3 Monaten um weniger als 8 %, insbesondere von 8 % bis 0,1 %, bevorzugt 5 % bis 3 % abnimmt.

3. Verfahren nach Anspruch 1 oder 2, **dadurch gekennzeichnet, dass** das permanent polarisierte Hydroxyapatit durch ein Verfahren erhalten wird, das die Schritte umfasst
(a) Bereiten von Proben aus Hydroxyapatit,
(b) Sintern der in Schritt (a) vorbereiteten Proben bei einer Temperatur zwischen 700 °C und 1200 °C,
(c) Anlegen einer konstanten oder variablen Gleichspannung zwischen 250 V und 2500 V über mindestens eine Minute bei einer Temperatur zwischen 900 °C und 1200 °C oder
Anlegen eines äquivalenten elektrischen Felds zwischen 1,49 kV/cm und 15 kV/cm über mindestens eine Minute bei einer Temperatur zwischen 900 °C und 1200 °C oder
Anlegen einer elektrostatischen Entladung zwischen 2500 V und 1500000 V über weniger als 10 Minuten bei einer Temperatur zwischen 900 °C und 1200 °C oder
Anlegen eines äquivalenten elektrischen Felds zwischen 148,9 kV/cm und 8928 kV/cm über weniger als 10 Minuten bei einer Temperatur zwischen 900 °C und 1200 °C und
(d) Abkühlen der in Schritt (c) erhaltenen Proben unter Beibehaltung der Gleichspannung oder des äquivalenten elektrischen Felds oder
Abkühlen der in Schritt (c) erhaltenen Proben unter Beibehaltung oder ohne Beibehaltung der elektrostatischen Entladung oder des äquivalenten elektrischen Felds.

4. Verfahren nach einem der vorhergehenden Ansprüche, **dadurch gekennzeichnet, dass** das permanent polarisierte Hydroxyapatit durch ein Verfahren erhalten wird, das die Schritte umfasst
(a) Bereiten von Proben aus Hydroxyapatit,
(b) Sintern der in Schritt (a) vorbereiteten Proben bei einer Temperatur von 1000 °C, insbesondere über 2 h,
(c) Anlegen eines äquivalenten elektrischen Felds von 3 kV/cm bei einer Temperatur von 1000 °C, insbesondere über 1 h, und
(d) Abkühlen der in Schritt (c) erhaltenen Proben unter Beibehaltung des äquivalenten elektrischen Felds, insbesondere über 30 Minuten.

5. Verfahren nach einem der vorhergehenden Ansprüche, **dadurch gekennzeichnet, dass** der Schritt zum in Kontakt bringen in Gegenwart von flüssigem Wasser ausgeführt wird.

6. Verfahren nach einem der vorhergehenden Ansprüche, **dadurch gekennzeichnet, dass** der Schritt zum in Kontakt bringen bei einem Volumenverhältnis von permanent polarisiertem Hydroxyapatit zu Wasser, insbesondere flüssigem Wasser, von 1000:1 bis 0,01:1, insbesondere 500:1 bis 100:1, bevorzugt 300:1 bis 350:1, ausgeführt wird.

7. Verfahren nach einem der vorhergehenden Ansprüche, **dadurch gekennzeichnet, dass** der Schritt zum in Kontakt bringen bei einem Volumenverhältnis von Kohlendioxid zu Methan von 200:1, insbesondere 3:1, bevorzugt 1:1, ausgeführt wird.

8. Verfahren nach einem der vorhergehenden Ansprüche, **dadurch gekennzeichnet, dass** der Schritt zum in Kontakt bringen unter einem Gesamtdruck von 0,1 bar bis 100 bar, insbesondere 1 bar bis 10 bar, bevorzugt von 6 bar, ausgeführt wird.

9. Verfahren nach einem der vorhergehenden Ansprüche, **dadurch gekennzeichnet, dass** der Schritt zum in Kontakt bringen unter einem Partialdruck von Kohlendioxid von 0,035 bar bis 90 bar, insbesondere 1 bar bis 3 bar, bevorzugt von 3 bar, und/oder unter einem Partialdruck von Methan von 0,00017 bar bis 5 bar, insbesondere 1 bar bis 3 bar, bevorzugt von 3 bar, ausgeführt wird.

10. Verfahren nach einem der vorhergehenden Ansprüche, **dadurch gekennzeichnet, dass** der Schritt zum in Kontakt bringen unter UV-Bestrahlung oder UV-Vis-Bestrahlung mit einer Wellenlänge von 200 nm bis 850 nm, insbesondere 240 nm bis 400 nm, bevorzugt 250 nm bis 260 nm, besonders bevorzugt von 253,7 nm, ausgeführt wird.

11. Verfahren nach einem der vorhergehenden Ansprüche, **dadurch gekennzeichnet, dass** der Schritt zum in Kontakt bringen unter UV-Bestrahlung und/oder Bestrahlung mit sichtbarem Licht, insbesondere mit einer Bestrahlungsstärke von 0,1 W/m² bis 200 W/m², insbesondere 1 W/m² bis 50 W/m², bevorzugt 2 W/m² bis 10 W/m², ausgeführt wird.

12. Verfahren nach einem der vorhergehenden Ansprüche, **dadurch gekennzeichnet, dass** der Schritt zum in Kontakt bringen bei einer Temperatur von 25 °C bis 250 °C, insbesondere 95 °C bis 140 °C, bevorzugt bei 95 °C, ausgeführt wird.

13. Verfahren nach einem der Ansprüche 1 bis 10, **dadurch gekennzeichnet, dass** der Schritt zum in Kontakt bringen ohne UV-Bestrahlung und bei einer Temperatur von 140 °C ausgeführt wird.

14. Verfahren nach einem der vorhergehenden Ansprüche, **dadurch gekennzeichnet, dass** der Schritt zum in Kontakt bringen kontinuierlich oder diskontinuierlich und/oder über 0,0001 h bis 120 h, insbesondere 24 h bis 72 h, bevorzugt 48 h bis 72 h, ausgeführt wird.

15. Verwendung eines Verfahrens nach einem der vorhergehenden Ansprüche zum Entfernen von Kohlendioxid aus der Atmosphäre.

## Revendications

1. Procédé pour la production, en particulier pour la production de manière sélective, d'éthanol comprenant l'étape de
- mise en contact d'un mélange de gaz comprenant ou constitué de dioxyde de carbone et de méthane en présence d'eau avec un catalyseur comprenant ou constitué d'hydroxyapatite polarisée de manière permanente.

2. Procédé selon la revendication 1, **caractérisé en ce que** l'hydroxyapatite polarisée de manière permanente possède
- une cristallinité > 65 %, en particulier > 70 %, préférablement > 75 % plus préférablement de 65 % à 99 %, et/ou
- une proportion de phosphate de calcium amorphe < 18 %, en particulier de 0,1 % à 17%, sur la base du poids total de l'hydroxyapatite polarisée de manière permanente, et/ou
- une proportion de phosphate de β-tricalcique < 36 %, en particulier de 0,1 % à 35 %, sur la base du poids total de l'hydroxyapatite polarisée de manière permanente, et/ou
- une résistance volumique de 10⁷ Ωcm² à 10⁵ Ωcm², en particulier de 10⁶ Ωcm² à 10⁵ Ωcm², la résistance volumique augmentant préférablement de seulement 4 % à 73 %, en particulier de 4 % à 63 %, préférablement de 4 %, après 3 mois, et/ou
- une capacité de surface diminuant de moins de 8 %, en particulier de 8 % à 0,1 %, préférablement de 5 % à 3 %, après 3 mois.

3. Procédé selon la revendication 1 ou 2, **caractérisé en ce que** l'hydroxyapatite polarisée de manière permanente est obtenue par un procédé comprenant les étapes de
(a) préparation d'échantillons d'hydroxyapatite,
(b) frittage des échantillons préparés dans l'étape (a) à une température entre 700 °C et 1 200 °C,
(c) application d'une tension continue constante ou variable comprise entre 250 V et 2 500 V pendant au moins 1 minute à une température entre 900 °C et 1 200 °C ou
application d'un champ électrique équivalent entre 1,49 kV/cm et 15 kV/cm pendant au moins 1 minute à une température entre 900 °C et 1 200 °C ou
application d'une décharge électrostatique entre 2 500 V et 1 500 000 V pendant moins de 10 minutes à une température entre 900 °C et 1 200 °C ou
application d'un champ électrique équivalent entre 148,9 kV/cm et 8 928 kV/cm pendant moins de 10 minutes à une température entre 900 °C et 1 200 °C, et
(d) refroidissement des échantillons obtenus dans l'étape (c) en maintenant la tension continue ou le champ électrique équivalent ou
refroidissement des échantillons obtenus dans l'étape (c) en maintenant ou sans maintenir la décharge électrostatique ou le champ électrique équivalent.

4. Procédé selon l'une quelconque des revendications précédentes, **caractérisé en ce que** l'hydroxyapatite polarisée de manière permanente est obtenue par un procédé comprenant les étapes de
(a) préparation d'échantillons d'hydroxyapatite,
(b) frittage des échantillons préparés dans l'étape (a) à une température de 1 000 °C, en particulier pendant 2 h,
(c) application d'un champ électrique équivalent de 3 kV/cm à une température de 1 000 °C, en particulier pendant 1 h, et
(d) refroidissement des échantillons obtenus dans l'étape (c) en maintenant le champ électrique équivalent, en particulier pendant 30 minutes.

5. Procédé selon l'une quelconque des revendications précédentes, **caractérisé en ce que** l'étape de mise en contact est mise en œuvre en la présence d'eau liquide.

6. Procédé selon l'une quelconque des revendications précédentes, **caractérisé en ce que** l'étape de mise en contact est mise en œuvre avec un rapport volumétrique de l'hydroxyapatite polarisée de manière permanente sur l'eau, en particulier l'eau liquide, de 1 000 : 1 à 0,01 : 1, en particulier de 500 : 1 à 100 : 1, préférablement de 300 : 1 à 350 : 1.

7. Procédé selon l'une quelconque des revendications précédentes, **caractérisé en ce que** l'étape de mise en contact est mise en œuvre avec un rapport volumétrique de dioxyde de carbone sur méthane de 200 : 1, en particulier de 3 : 1, préférablement de 1 : 1.

8. Procédé selon l'une quelconque des revendications précédentes, **caractérisé en ce que** l'étape de mise en contact est mise en œuvre sous une pression totale de 0,1 bar à 100 bars, en particulier de 1 bar à 10 bars, préférablement de 6 bars.

9. Procédé selon l'une quelconque des revendications précédentes, **caractérisé en ce que** l'étape de mise en contact est mise en œuvre sous une pression partielle de dioxyde de carbone de 0,035 bar à 90 bars, en particulier de 1 bar à 3 bars, préférablement de 3 bars, et/ou sous une pression partielle de méthane de 0,00017 bar à 5 bars, en particulier de 1 bar à 3 bars, préférablement de 3 bars.

10. Procédé selon l'une quelconque des revendications précédentes, **caractérisé en ce que** l'étape de mise en contact est mise en œuvre sous une irradiation UV ou sous une irradiation UV-Vis possédant une longueur d'onde de 200 nm à 850 nm, en particulier de 240 nm à 400 nm, préférablement de 250 nm à 260 nm, plus préférablement de 253,7 nm.

11. Procédé selon l'une quelconque des revendications précédentes, **caractérisé en ce que** l'étape de mise en contact est mise en œuvre sous une irradiation UV et/ou une irradiation par de la lumière visible, en particulier possédant une irradiance de 0,1 W/m² à 200 W/m², en particulier de 1 W/m² à 50 W/m², préférablement de 2 W/m² à 10 W/m².

12. Procédé selon l'une quelconque des revendications précédentes, **caractérisé en ce que** l'étape de mise en contact est mise en œuvre à une température de 25 °C à 250 °C, en particulier de 95 °C à 140 °C, préférablement de 95 °C.

13. Procédé selon l'une quelconque des revendications 1 à 10, **caractérisé en ce que** l'étape de mise en contact est mise en œuvre sans irradiation UV et à une température de 140 °C.

14. Procédé selon l'une quelconque des revendications précédentes, **caractérisé en ce que** l'étape de mise en contact est mise en œuvre de manière continue ou de manière discontinue et/ou pendant 0,0001 h à 120 h, en particulier de 24 h à 72 h, préférablement de 48 h à 72 h.

15. Utilisation d'un procédé selon l'une quelconque des revendications précédentes pour l'élimination de dioxyde de carbone de l'atmosphère.
